# EUROPÄISCHE PATENTSCHRIFT

(11) **EP 3 538 460 B1**
(45) Veröffentlichungstag und Bekanntmachung des Hinweises auf die Patenterteilung: **23.12.2020**
(21) Anmeldenummer: 17800365.3
(22) Anmeldetag: 06.11.2017
(51) Int. Cl.: B65G 51/06

(54) **ROHRPOSTHÜLSE**
PNEUMATIC-TUBE CONTAINER
CARTOUCHE DE COURRIER PAR TUBE

(30) Priorität: 08.11.2016 AT 510182016
(43) Veröffentlichungstag der Anmeldung: 18.09.2019
(73) Patentinhaber: Ing. Sumetzberger GmbH, 1110 Wien (AT)
(72) Erfinder: FRIEDRICH, Peter, 1040 Wien (AT)
(74) Vertreter: Müllner, Martin
(86) Internationale Anmeldenummer: PCT/AT2017/060294
(87) Internationale Veröffentlichungsnummer: WO 2018/085871

(56) Entgegenhaltungen:
- EP-A1- 0 706 961
- EP-A1- 2 657 160
- EP-A2- 0 761 576
- WO-A1-2013/056281

## Beschreibung

### Technisches Gebiet

Die vorliegende Erfindung betrifft eine Rohrposthülse mit einem Hülsenrohr und mit einem Deckel bzw. einem Boden an den beiden Enden des Hülsenrohrs, wobei zumindest der Deckel von einer Geschlossenstellung in eine Offenstellung bewegbar ist, wobei im Hülsenrohr ein Einsatz vorgesehen ist, der zur Aufnahme von Proberöhrchen zumindest einen rohrförmigen Kanal aufweist, der sich parallel zur Längsachse des Hülsenrohres erstreckt, wobei in zumindest einem Kanal zumindest ein Klemmelement vorgesehen ist, das seitlich in den Kanal hineinragt.

### Stand der Technik

Rohrposthülsen werden in Spitälern oft zum Transport von Blutproben, die sich in Proberöhrchen befinden, eingesetzt. Der Transport solcher Proberöhrchen stellt besondere Anforderungen. Zum einen können solche Proberöhrchen brechen, wenn zwei Proberöhrchen aufeinander aufschlagen, da die Kräfte beim Rohrposttransport doch erheblich sind. Andererseits kommt es bei stärkeren Erschütterungen - selbst wenn die Proberöhrchen nicht brechen - zu einer Hämolyse im Blut, was die Blutprobe unbrauchbar macht. (Bei einer Hämolyse brechen die roten Blutkörperchen, sodass sich deren Inhaltsstoffe mit dem Blutplasma vermischen und so die Werte des Blutplasmas verfälschen.)

Es sind daher schon verschiedene Vorschläge gemacht worden, wie man die Proberöhrchen in der Rohrposthülse fixieren kann, sodass sie nicht gegen die Wand der Rohrposthülse oder gegeneinander schlagen können. Eine derartige Lösung ist in AT 12017 U1 beschrieben. Es wird dort ein Einsatz verwendet, der ein zur Rohrposthülse koaxiales Gestell besitzt, das aus einer Trägersäule mit zumindest einem Boden und Halterungen, die die Trägersäule ringförmig umschließen, in mehreren Ebenen besteht. Die Halterungen bestehen aus Ringen mit Fortsätzen in radialer Richtung, wobei Proberöhrchen zwischen benachbarten Fortsätzen eingeklemmt werden können. Es können vier solcher Halterungen übereinander vorgesehen sein, sodass zwei Proberöhrchen übereinander angebracht werden können, die jeweils an zwei Stellen gehalten werden.

Nachteilig an dieser Lösung ist, dass diese Halterung nicht einfach beschickt oder entladen werden kann, wenn sie nicht aus der Rohrposthülse herausgenommen wird. Damit eignet sie sich nur schlecht für ein automatisches Beschicken und ein automatisches Entladen.

Aus der gattungsbildenden EP 2657160 A ist bekannt, in der Rohrposthülse Kanäle vorzusehen, in welche die Proberöhrchen eingeschoben werden können. Die Kanäle bieten dabei für zumindest zwei Proberöhrchen hintereinander Platz. Damit nun ein einzelnes Proberöhrchen nicht im Kanal hin und her rutschen kann (was Hämolyse zur Folge haben kann), sind in jedem Kanal zumindest zwei Klemmelemente vorgesehen, die seitlich in den Kanal hineinragen, sodass auch ein einzelnes Proberöhrchen sicher geklemmt werden kann, egal, an welcher Position im Kanal es sich befindet.

Diese Lösung hat zwar den Vorteil, dass die Proberöhrchen eingeschoben und entnommen werden können, ohne dass das Element mit den Kanälen aus der Rohrposthülse herausgenommen werden muss; zum Entladen ist es aber notwendig, dass die Rohrposthülse auf beiden Seiten geöffnet wird und dass man mit einem Ausschieber, der eine der Anzahl der Kanäle entsprechende Anzahl von Stäben hat, in die Rohrposthülse von der einen Seite hineinfährt, sodass die Proberöhrchen auf der anderen Seite herausgeschoben werden. Auch dies ist relativ aufwändig.

Aus EP 706961 A1 ist eine Rohrposthülse mit einem Hülsenrohr und mit einem Deckel bzw. einem Boden an den beiden Enden des Hülsenrohrs bekannt, wobei der Deckel und der Boden von einer Geschlossenstellung in eine Offenstellung bewegbar sind. Im Hülsenrohr sind Klemmelemente, z.B. zum Klemmen von Geldscheinen, vorgesehen, deren Bewegung mit der Bewegung des Deckels bzw. des Bodens aus der Geschlossenstellung in die Offenstellung bzw. aus der Offenstellung in die Geschlossenstellung gekoppelt ist. Anders gesagt offenbart EP 0706961 A1 eine Rohrposthülse mit einem Hülsenrohr und mit einem Deckel bzw. einem Boden an den beiden Enden des Hülsenrohrs, wobei zumindest der Deckel von einer Geschlossenstellung in eine Offenstellung bewegbar ist, wobei im Hülsenrohr zumindest ein rohrförmiger Kanal angeordnet ist, der sich parallel zur Längsachse des Hülsenrohres erstreckt, wobei in zumindest einem Kanal zumindest ein Klemmelement vorgesehen ist, dessen Bewegungen mit der Bewegung des Deckels aus der Geschlossenstellung in die Offenstellung bzw. aus der Offenstellung in die Geschlossenstellung gekoppelt ist.

### Darstellung der Erfindung

Es ist Aufgabe der vorliegenden Erfindung, eine Rohrposthülse der eingangs genannten Art zu schaffen, die einfach mit Proberöhrchen beschickt werden kann und aus der die Proberöhrchen einfach entnommen werden können, und bei der dennoch keine Gefahr von Hämolyse gegeben ist.

Diese Aufgabe wird durch eine Rohrposthülse der eingangs genannten Art erfindungsgemäß dadurch gelöst, dass das Klemmelement aus dem Kanal heraus bewegbar ist und dass diese Bewegung aus dem Kanal heraus bzw. in diesen hinein mit der Bewegung des Deckels aus der Geschlossenstellung in die Offenstellung bzw. aus der Offenstellung in die Geschlossenstellung gekoppelt ist.

Auf diese Weise braucht die Rohrposthülse nur an einer Seite geöffnet zu werden, damit sich die Klemmelemente lösen, und die Proberöhrchen fallen heraus, wenn die geöffnete Seite der Rohrposthülse nach unten gehalten wird. Solch eine Rohrposthülse lässt sich also genauso leicht automatisch entleeren wie eine Rohrposthülse, in der die Proberöhrchen ungesichert transportiert werden. Da die Proberöhrchen aber während des Transports der Rohrposthülse geklemmt sind, ist dennoch keine Gefahr von Hämolyse gegeben.

Es sind zwei Arten von Deckeln von Rohrposthülsen verbreitet. Bei einer Art besteht der Deckel aus mehreren (drei) Sektoren, die an einem Betätigungsring angelenkt sind und die durch Verschieben des Betätigungsrings aus der Geschlossenstellung in die Offenstellung bewegbar sind. Solch eine Rohrposthülse ist z.B. aus EP 697354 A bekannt. Bei solch einem Deckel lässt sich die vorliegende Erfindung dadurch realisieren, dass das Klemmelement bzw. die Klemmelemente an einem Rohr angebracht sind, welches koaxial zum Hülsenrohr in diesem angebracht ist, dass dieses Rohr um seine Längsachse drehbar ist, und dass das Rohr zum Erzielen einer Kulissenführung einen schräg verlaufenden Schlitz aufweist, in den ein Stift des drehfest geführten Betätigungsringes eingreift, sodass eine Verschiebung des Betätigungsringes eine Drehung des Rohrs bewirkt. Das Klemmelement ist dabei elastisch ausgebildet und ragt bei geschlossenem Deckel in den Kanal, und wenn sich ein Proberöhrchen an dieser Stelle befindet, drückt das Klemmelement unter elastischer Deformation gegen das Proberöhrchen und klemmt es fest. Wenn das Rohr beim Öffnen des Deckels verdreht wird, schwenkt das Klemmelement aus dem Kanal heraus und gibt das Proberöhrchen frei.

Diese Lösung lässt sich auch dann realisieren, wenn auch der Boden mehrere Bodensektoren aufweist, die an einem Betätigungsring angelenkt sind und die durch Verschieben des Betätigungsrings aus der Geschlossenstellung in die Offenstellung bewegbar sind, also wenn die Rohrposthülse an beiden Enden in gleicher Weise geöffnet werden kann. In diesem Fall sieht man vor, dass das Rohr für jeden Betätigungsring einen schräg verlaufenden Schlitz aufweist, in den jeweils ein Stift des entsprechenden Betätigungsringes eingreift, dass die beiden schräg verlaufenden Schlitze gleichsinnig geneigt sind und dass jeder schräg verlaufende Schlitz an seinem dem jeweiligen Ende des Hülsenrohrs zugewandten Ende in einen Schlitz übergeht, der in Umfangsrichtung des Rohrs verläuft, sodass durch Öffnen von nur entweder dem Deckel oder dem Boden das Klemmelement bzw. die Klemmelemente aus dem jeweiligen Kanal in jeweils verschiedener Richtung hinausbewegt werden.

Andererseits sind auch Rohrposthülsen bekannt, bei denen der Deckel um eine zur Längsachse des Hülsenrohrs parallele Achse verschwenkbar ist. Solch eine Rohrposthülse ist in der bereits erwähnten EP 2657160 A dargestellt. Bei solch einer Rohrposthülse lässt sich die vorliegende Erfindung realisieren, indem das Klemmelement bzw. die Klemmelemente an einem Rohr angebracht sind, welches koaxial zum Hülsenrohr in diesem angebracht ist, indem dieses Rohr um seine Längsachse drehbar ist und indem die Drehbewegung des Rohrs mit der Verschwenkbewegung des Deckels gekoppelt ist. Diese Kopplung kann ganz einfach durch eine entsprechende Verzahnung erfolgen.

### Kurze Beschreibung der Zeichnungen

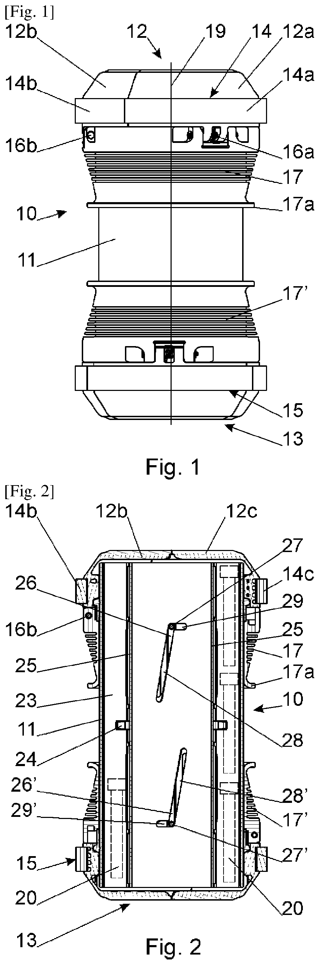

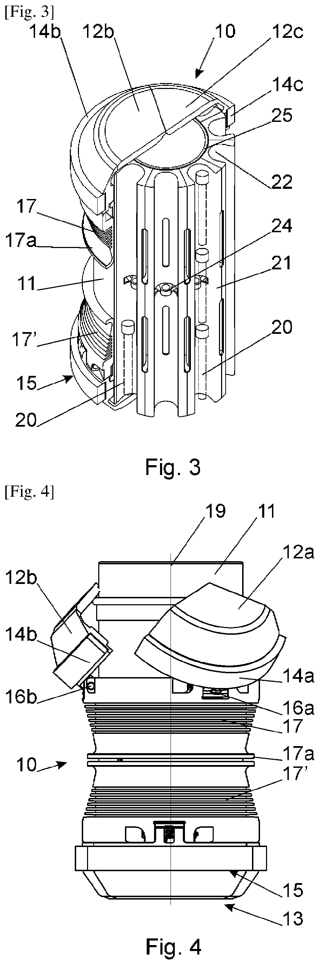

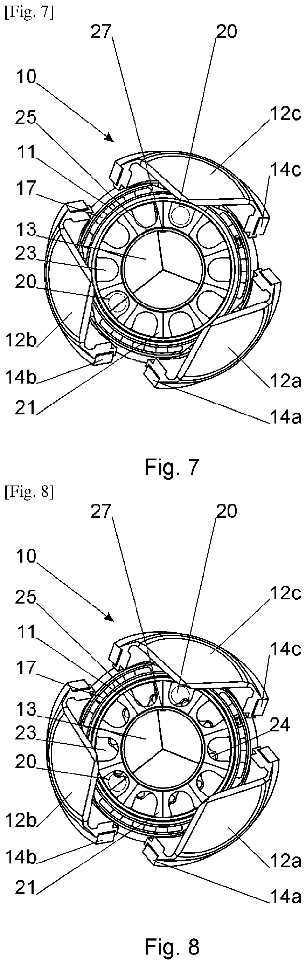

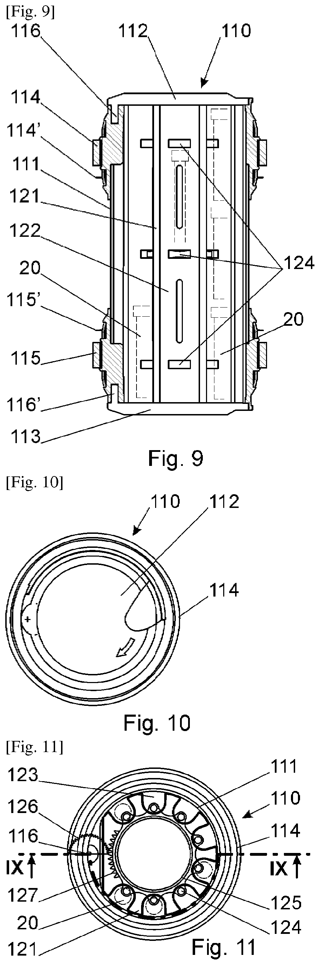

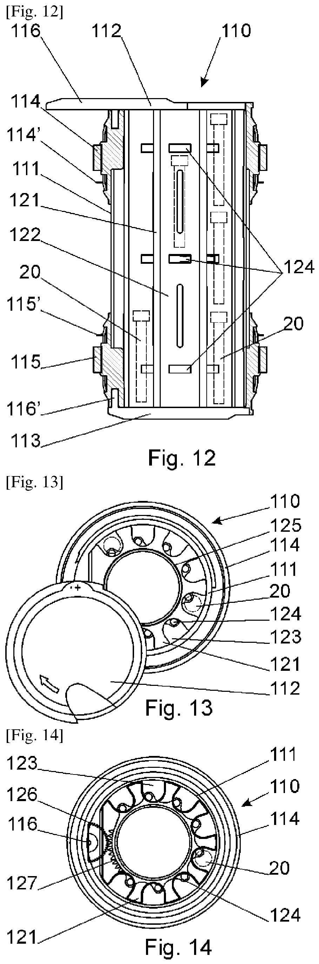

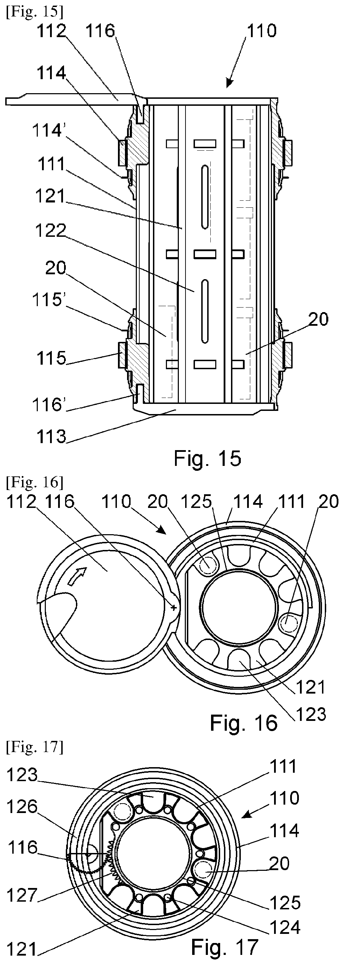

An Hand der beiliegenden Zeichnungen wird die vorliegende Erfindung näher erläutert. Es zeigt: Fig. 1 eine erfindungsgemäße Rohrposthülse einer ersten Ausführungsform in geschlossener Stellung von der Seite; Fig. 2 dieselbe im Längsschnitt; Fig. 3 dieselbe in perspektivischer Ansicht, wobei das Hülsenrohr und die beiden Hülsenköpfe zur Hälfte weggebrochen sind; Fig. 4 bis 6 zeigen diese Rohrposthülse in entsprechenden Ansichten wie bei den Fig. 1 bis 3, aber in geöffneter Stellung; Fig. 7 zeigt diese Rohrposthülse in geöffneter Stellung von oben; Fig. 8 zeigt diese Rohrposthülse in halb geöffneter Stellung von oben; Fig. 9 zeigt eine Rohrposthülse einer zweiten Ausführungsform in geschlossener Stellung in einem Schnitt entlang der Linie IX-IX von Fig. 11; Fig. 10 dieselbe von oben; Fig. 11 dieselbe von oben, jedoch mit entferntem Deckel; die Fig. 12 bis 14 zeigen dieselbe Hülse in entsprechenden Ansichten wie bei den Fig. 8 bis 10, jedoch in halb geöffneter Stellung; und die Fig. 15 bis 17 zeigen dieselbe Hülse in entsprechenden Ansichten wie bei den Fig. 8 bis 10, jedoch in ganz geöffneter Stellung.

### Weg(e) zur Ausführung der Erfindung

Die Rohrposthülse 10 weist - wie üblich - ein Hülsenrohr 11, einen Deckel 12 und einen Boden 13 auf; am Deckel 12 ist ein Gleitring 14 und am Boden 13 ist ein Gleitring 15 angebracht. Wenn sich die Rohrposthülse 10 in einem Fahrrohr der Rohrpostanlage befindet, liegen die Gleitringe 14, 15 dicht am Fahrrohr an, sodass die Rohrposthülse 10 im Fahrrohr geführt wird und durch eine Druckdifferenz im Fahrrohr transportiert werden kann.

Der Deckel 12 ist aus drei Deckelsektoren 12a bis 12c zusammengesetzt. Auch der Gleitring 14 besteht demgemäß aus drei Teilen 14a bis 14c, wobei jeder Deckelsektor 12a bis 12c einen entsprechenden Teil 14a bis 14c des Gleitringes 14 trägt. Jeder der Deckelsektoren 12a bis 12c ist mittels einer Drehachse an einem Betätigungsring 17 schwenkbar gelagert, wobei in den Figuren nur die Drehachse 16a und 16b der Deckelsektoren 12a und 12b sichtbar sind. Der Betätigungsring 17 ist um das Hülsenrohr 11 herum angeordnet und lässt sich in Richtung der Hülsenlängsachse 19 verschieben. Daher befinden sich auch die Drehachsen 16a, 16b der drei Deckelsektoren 12a, 12b, 12c knapp außerhalb des Hülsenrohrs 11. Sie verlaufen außerdem normal zur Hülsenlängsachse 19 und tangential zum Hülsenrohr 11. Die Deckelsektoren 12a bis 12c lassen sich daher um die Drehachsen 16a, 16b in eine Öffnungsstellung schwenken, und durch eine Verschiebung des Betätigungsringes 17 können sie außen am Hülsenrohr 11 vorbeigleiten (siehe Fig. 4 bis 7).

Der Betätigungsring 17 weist einen Vorsprung 17a auf, sodass derartige Rohrposthülsen 10 in einfacher Weise automatisch geöffnet werden können: Ein Greifer der Rohrpoststation kommt an dem Vorsprung 17a zum Anliegen, und wenn nun entweder der Greifer bewegt und die Rohrposthülse 10 festgehalten wird oder wenn die Rohrposthülse 10 gegenüber dem feststehenden Greifer bewegt wird, wird der Gleitring 17 verschoben und die Hülse geöffnet.

Derartige Rohrposthülsen 10 können auch in einfacher Weise selbstschließend ausgebildet werden, indem die Deckelsektoren 12a bis 12c federbelastet in Richtung Geschlossenstellung ausgeführt werden.

Damit mit einer derartigen Rohrposthülse 10 Proberöhrchen 20 transportiert werden können, ist in das Hülsenrohr 11 ein Einsatz 21 eingesetzt. Dieser Einsatz 21 hat nutartige Vertiefungen 22, die zusammen mit dem Hülsenrohr 11 Kanäle 23 begrenzen, in welche die Proberöhrchen 20 eingeschoben werden können. Im Ausführungsbeispiel sind zehn derartige Kanäle 23 vorgesehen, die jeweils drei kurze Proberöhrchen 20 aufnehmen können, sodass insgesamt bis zu dreißig Proberöhrchen transportiert werden können. Alternativ dazu kann jeder Kanal 23 zwei lange Proberöhrchen aufnehmen.

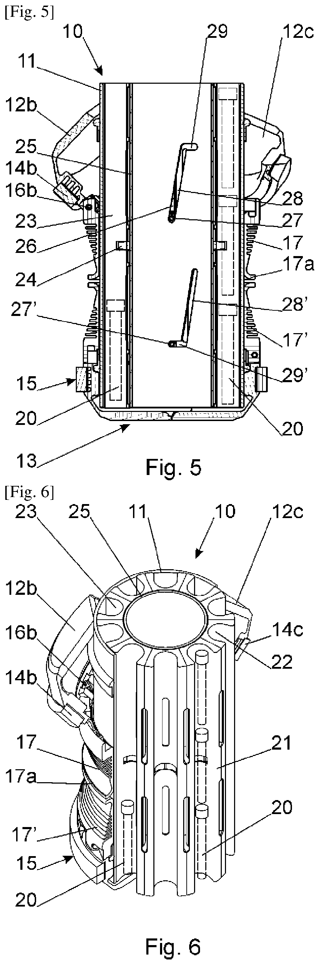

Solange in jedem Kanal 23 drei kurze oder zwei lange Proberöhrchen eingesetzt sind, ist deren Bewegungsspielraum gering. Wenn aber nur ein Proberöhrchen 20 eingesetzt ist (in Fig. 2 und 5 links dargestellt), gibt es allerdings folgendes Problem: Wenn die Rohrposthülse 10 nach oben transportiert und dann abgebremst wird, bewegt sich das Proberöhrchen 20 zunächst gleichförmig weiter, also in Bezug auf die Rohrposthülse 10 nach oben, bis es am Deckel 12 aufschlägt. Auf dieser Strecke von rund 15-20 cm wurde die Rohrposthülse 10 aber schon relativ stark abgebremst, sodass die Geschwindigkeitsdifferenz zwischen Proberöhrchen 20 und Deckel 12 beim Aufschlag relativ groß ist. Unmittelbar nach dem Aufschlag fällt das Proberöhrchen 20 dann wieder nach unten und schlägt neuerlich auf. Dadurch kann es in ungünstigen Fällen zu Hämolyse kommen, wodurch die Blutprobe unbrauchbar wird. Wird die Rohrposthülse 10 nach unten transportiert, gibt es ein ähnliches Problem beim Beschleunigen.

Um Hämolyse zu verhindern, ist nun in jedem Kanal 23 ein Klemmelement 24 aus elastischem Material vorgesehen. Wenn drei Proberöhrchen 20 eingesetzt sind (rechts in Fig. 2 und 5), drückt dieses Klemmelement 24 unter entsprechender Verformung gegen das mittlere Proberöhrchen. Wenn nur ein Proberöhrchen eingesetzt ist (links in Fig. 2 und 5), dann ragt das Klemmelement 24 in den Kanal, sodass sich das Proberöhrchen 20 nur um wenige Zentimeter bewegen kann und außerdem wegen der Elastizität des Materials des Klemmelements 24 relativ weich aufschlägt.

Infolge der Klemmelemente 24 fallen aber nun nicht mehr alle Proberöhrchen 20 aus der Rohrposthülse 10 heraus, wenn man sie mit der Öffnung nach unten hält. Aus diesem Grunde ist erfindungsgemäß vorgesehen, dass die Klemmelemente 24 auf einem Rohr 25 angebracht sind, welches koaxial zum Hülsenrohr 11 ist und bezüglich dieses Hülsenrohrs 11 drehbar, aber axial unverschiebbar gelagert ist. Verdreht man das Rohr 25, schwenken die Klemmelemente 24 seitlich aus der Nut 22 heraus (vergleiche Fig. 3 mit Fig. 6 sowie Fig. 7 mit Fig. 8). Sobald die Klemmelemente 24 aus den jeweiligen Nuten 22 herausgeschwenkt sind, fallen alle Proberöhrchen 20 nur durch Schwerkrafteinwirkung aus der Rohrposthülse heraus.

Damit nun dieses Verdrehen des Rohrs 25 automatisch beim Öffnen der Rohrposthülse 10 geschieht, ist im Hülsenrohr 11 ein achsparalleler Schlitz 26 vorgesehen (nur in den Fig. 2 und 5 zu sehen, und auch dort nur teilweise, hinter dem gleich erläuterten Schlitz 28), den ein in den Betätigungsring 17 eingesetzter Stift 27 durchgreift. Beim Öffnen und Schließen der Rohrposthülse 10 gleitet der Stift 27 in diesem Schlitz 26 nach unten bzw. oben. Im Rohr 25 ist ein schräg verlaufender Schlitz 28 vorgesehen, in den der Stift 27 ebenfalls eingreift. Dadurch ergibt sich eine Kulissenführung, die beim Öffnen und Schließen der Rohrposthülse 10 eine entsprechende Drehung des Rohrs 25 bewirkt. M.a.W.: Bei geöffneter Rohrposthülse 10 sind die Klemmelemente 24 aus den Kanälen 23 herausgeschwenkt, bei geschlossener Rohrposthülse 10 sind sie eingeschwenkt.

Natürlich ist es notwendig, dass der Einsatz 21 einen mit dem Schlitz 26 im Hülsenrohr 11 fluchtenden Schlitz (oder an dieser Stelle eine Öffnung) hat, damit der Stift 27 nicht durch den Einsatz 21 in seiner Bewegung gehindert ist.

Rohrposthülsen 10 sind oft symmetrisch ausgeführt, d.h. der Boden 13 ist gleich wie der Deckel 12 ausgebildet. Auch der Boden 13 besteht also aus drei Segmenten, die an einem Betätigungsring 17' gelagert sind. Damit nun auch beim Öffnen des Bodens 13 die Klemmelemente 24 ausschwenken, ist ein weiterer Schlitz 26' (nur in Fig. 2 zu sehen und auch dort nur teilweise, hinter dem gleich erläuterten Schlitz 28') im Hülsenrohr 11 vorgesehen, den ein Stift 27' des Betätigungsrings 17' des Bodens 13 durchgreift. Dieser Stift 27' greift in einen weiteren, schräg verlaufenden Schlitz 28' des Rohrs 25 ein, sodass sich dieses bei Öffnen des Bodens 13 verdreht. Damit nicht die Kulissenführung des Deckels 12 diese Bewegung blockiert, geht der schräg verlaufende Schlitz 26 an seinem oberen Ende in einen waagrechten (also in Umfangsrichtung verlaufenden) Schlitz 29 über. Analog dazu geht der schräg verlaufende Schlitz 28' in einen waagrechten Schlitz 29' über, damit der Boden 13 nicht die Öffnungsbewegung des Deckels 12 blockiert.

In den Fig. 9 bis 17 ist eine Rohrposthülse 110 mit einem Hülsenrohr 111 gezeigt, das einen Deckel 112 aufweist, der um eine Achse 116, die sich am Rand der Rohrposthülse befindet, wegschwenkbar ist (vgl. Fig. 9, 12 und 15; 10, 13 und 16; sowie 11, 14 und 17). Ein Boden 113 ist analog dazu um eine Achse 116' wegschwenkbar. Unabhängig von Deckel 112 und Boden 113 sind Gleitringe 114 und 115 am Hülsenrohr 111 vorgesehen, die Abdichtung gegen das Hülsenrohr erfolgt hier mit Dichtringen 114' und 115'.

Ebenso wie bei der ersten Ausführungsform ist ein Einsatz 121 im Hülsenrohr 111 vorgesehen, dessen Nuten 122 zusammen mit dem Hülsenrohr 111 Kanäle 123 bildet, in die Proberöhrchen 20 eingeschoben werden können. Ebenso ist ein Rohr 125 vorgesehen, das hier allerdings für jeden Kanal 123 drei Klemmelemente 124 in verschiedenen Höhen aufweist. Somit werden auch einzelne kurze Proberöhrchen geklemmt (Fig. 9, linker Kanal). Das Rohr 125 ist wiederum drehbar gelagert. Der Antrieb erfolgt über ein Zahnrad 126, das in einen entsprechenden Zahnring 127 des Rohrs 125 eingreift.

Wenn - wie dargestellt - das Hülsenrohr symmetrisch ist, dann muss für den Boden 113 ein entsprechendes Zahnrad vorgesehen sein, das in eine weitere Verzahnung des Rohrs 125 eingreift (nicht dargestellt). Damit die beiden Zahnräder einander nicht gegenseitig blockieren, müssen sie bei geschlossenem Deckel 112 bzw. bei geschlossenem Boden 113 an der entsprechenden Stelle eine Lücke in der Verzahnung aufweisen. Sie können auch - wie dargestellt - als halbe Zahnräder ausgeführt sein. Damit sich das Rohr 125 nicht unbeabsichtigt verdreht, wenn sowohl Boden 113 als auch Deckel 112 geschlossen sind und somit beide Zahnräder außer Eingriff stehen, sollte das Rohr 125 in dieser Stellung, also in der Klemmstellung, verrasten.

## Patentansprüche

1. Rohrposthülse (10, 110) mit einem Hülsenrohr (11, 111) und mit einem Deckel (12, 112) bzw. einem Boden (13, 113) an den beiden Enden des Hülsenrohrs (11, 111), wobei zumindest der Deckel (12, 112) von einer Geschlossenstellung in eine Offenstellung bewegbar ist, wobei im Hülsenrohr (11, 111) ein Einsatz (21, 121) vorgesehen ist, der zur Aufnahme von Proberöhrchen (20) zumindest einen rohrförmigen Kanal (23, 123) aufweist, der sich parallel zur Längsachse (19) des Hülsenrohres (11, 111) erstreckt, wobei in zumindest einem Kanal (23, 123) zumindest ein Klemmelement (24, 124) vorgesehen ist, das seitlich in den Kanal (23, 123) hineinragt, wobei das Klemmelement (24, 124) aus dem Kanal (23, 123) heraus bewegbar ist und dass diese Bewegung aus dem Kanal (23, 123) heraus bzw. in diesen hinein mit der Bewegung des Deckels (12, 112) aus der Geschlossenstellung in die Offenstellung bzw. aus der Offenstellung in die Geschlossenstellung gekoppelt ist.

2. Rohrposthülse (10) nach Anspruch 1, wobei der Deckel (12) mehrere Deckelsektoren (12a, 12b, 12c) aufweist, die an einem Betätigungsring (17) angelenkt sind und die durch Verschieben des Betätigungsringes (17) aus der Geschlossenstellung in die Offenstellung bewegbar sind, **dadurch gekennzeichnet, dass** das Klemmelement (24) bzw. die Klemmelemente (24) an einem Rohr (25) angebracht sind, welches koaxial zum Hülsenrohr (11) in diesem angebracht ist, dass dieses Rohr (25) um seine Längsachse (19) drehbar ist, und dass das Rohr (25) zum Erzielen einer Kulissenführung einen schräg verlaufenden Schlitz (28) aufweist, in den ein Stift (27) des drehfest geführten Betätigungsringes (17) eingreift, sodass eine Verschiebung des Betätigungsringes (17) eine Drehung des Rohrs (25) bewirkt.

3. Rohrposthülse (10) nach Anspruch 2, wobei auch der Boden (13) mehrere Bodensektoren aufweist, die an einem Betätigungsring (17') angelenkt sind und die durch Verschieben des Betätigungsringes (17') aus der Geschlossenstellung in die Offenstellung bewegbar sind, **dadurch gekennzeichnet, dass** das Rohr (25) für jeden Betätigungsring (17, 17') einen schräg verlaufenden Schlitz (28, 28') aufweist, in den jeweils ein Stift (27, 27') des entsprechenden Betätigungsrings (17, 17') eingreift, dass die beiden schräg verlaufenden Schlitze (28, 28') gleichsinnig geneigt sind und dass jeder schräg verlaufende Schlitz (28, 28') an seinem dem jeweiligen Ende des Hülsenrohrs (11) zugewandten Ende in einen Schlitz (29, 29') übergeht, der in Umfangsrichtung des Rohrs verläuft, sodass durch Öffnen von nur entweder dem Deckel (12) oder dem Boden (13) das Klemmelement (24) bzw. die Klemmelemente (24) aus dem jeweiligen Kanal (23) in jeweils verschiedener Richtung hinausbewegt werden.

4. Rohrposthülse (110) nach Anspruch 1, wobei der Deckel (112) um eine zur Längsachse des Hülsenrohrs (111) parallele Achse (116) verschwenkbar ist, **dadurch gekennzeichnet, dass** das Klemmelement (124) bzw. die Klemmelemente (124) an einem Rohr (125) angebracht sind, welches koaxial zum Hülsenrohr (111) in diesem angebracht ist, dass dieses Rohr (125) um seine Längsachse drehbar ist und dass die Drehbewegung des Rohrs (125) mit der Verschwenkbewegung des Deckels (112) gekoppelt ist.

## Claims

1. A pneumatic tube sleeve (10, 110) with a sleeve tube (11, 111) and having a lid (12, 112) and a bottom (13, 113) at the two ends of the sleeve tube (11, 111), respectively, wherein at least the lid (12, 112) is movable from a closed position into an open position, an insert (21, 121) being provided in the sleeve tube (11, 111) which for receiving sample tubes (20) includes at least one tubular channel (23, 123) extending in parallel to the longitudinal axis (19) of the sleeve tube (11, 111), in at least one channel (23, 123) at least one clamping element (24, 124) being provided projecting laterally into the channel (23, 123), wherein the clamping element (24, 124) is movable out of the channel (23, 123), and that this movement out of the channel (23, 123) and into the channel (23, 123), respectively, is coupled to the movement of the lid (12, 112) from the closed position into the open position and from the open position into the closed position, respectively.

2. The pneumatic tube sleeve (10) according to claim 1, wherein the lid (12) includes a plurality of lid sectors (12a, 12b, 12c) which are articulated to an actuating ring (17), and which are movable from the closed position into the open position by displacement of the actuating ring (17), **characterized in that** the clamping element (24) and the clamping elements (24), respectively, are attached to a tube (25) which is attached in the sleeve tube (11) coaxially to the sleeve tube (11), that this tube (25) is rotatable about its longitudinal axis (19), and that the tube (25) includes an obliquely extending slot (28) for achieving a slide guide into which a pin (27) of the non-rotationally guided actuating ring (17) is engaging such that a displacement of the actuating ring (17) causes a rotation of the tube (25).

3. The pneumatic tube sleeve (10) according to claim 2, wherein the bottom (13) includes a plurality of bottom sectors, too, which are articulated to an actuating ring (17'), and which are movable from the closed position into the open position by displacement of the actuating ring (17'), **characterised in that** for each actuating ring (17, 17') the tube (25) includes an obliquely extending slot (28, 28') into which a pin (27, 27') of the corresponding actuating ring (17, 17') is engaging each, that the two obliquely extending slots (28, 28') are inclined in the same sense, and that each obliquely extending slot (28, 28') transitions at its end facing the respective end of the sleeve tube (11) into a slot (29, 29') extending in the circumferential direction of the tube such that by opening only either the lid (12) or the bottom (13) the clamping element (24) and the clamping elements (24), respectively, are moved out of the respective channel (23) each in different directions.

4. The pneumatic tube sleeve (110) according to Claim 1, wherein the lid (112) is pivotable about an axis (116) in parallel to the longitudinal axis of the sleeve tube (111), **characterized in that** the clamping element (124) and the clamping elements (124), respectively, are attached to a tube (125) which is attached in the sleeve tube (111) coaxially to the sleeve tube (111), that this tube (125) is rotatable about its longitudinal axis, and that the rotational movement of the tube (125) is coupled to the pivoting movement of the lid (112).

## Revendications

1. Curseur pour systèmes de transport pneumatique (10, 110) pourvu d'un cylindre tubulaire de curseur (11, 111) et, sur les deux extrémités du cylindre tubulaire de curseur (11, 111), d'un couvercle (12, 112) et d'un fond (13, 113), au moins le couvercle (12, 112) pouvant être déplacé depuis une position de fermeture vers une position d'ouverture, l'intérieur du cylindre tubulaire de curseur (11, 111) étant pourvu d'un support (21, 121) comportant au moins un canal de forme tubulaire (23, 123) qui permet d'y loger des tubes d'échantillon (20) et qui s'étend parallèlement à l'axe longitudinal (19) du cylindre tubulaire de curseur (11, 111), l'au moins un canal (23, 123) étant pourvu d'au moins un élément de serrage (24, 124) qui s'étend latéralement dans le canal (23, 123), l'élément de serrage (24, 124) pouvant être retiré du canal (23, 123), un tel mouvement de retrait du canal (23, 123) et de mise en place dans ce dernier étant couplé à un déplacement du couvercle (12, 112) depuis la position de fermeture vers la position d'ouverture et depuis la position d'ouverture vers la position de fermeture, respectivement.

2. Curseur pour systèmes de transport pneumatique (10) selon la revendication 1, le couvercle (12) comportant plusieurs secteurs de couvercle (12a, 12b, 12c) lesquels sont articulés sur un anneau d'actionnement (17) et lesquels peuvent être déplacés en faisant glisser l'anneau d'actionnement (17) depuis la position de fermeture vers la position d'ouverture, **caractérisé en ce que** l'élément de serrage (24) ou les éléments de serrage (24) sont montés sur un tube (25) qui se trouve en disposition coaxiale au sein du cylindre tubulaire de curseur (11), que le tube (25) peut être tourné autour de son axe longitudinal (19), et que le tube (25) présente une fente (28) s'étendant en biais pour ainsi constituer une coulisse de guidage dans laquelle s'engage un ergot (27) de l'anneau d'actionnement (17) qui est guidé de manière solidaire en rotation, un déplacement de l'anneau d'actionnement (17) provoquant ainsi une rotation du tube (25).

3. Curseur pour systèmes de transport pneumatique (10) selon la revendication 2, le fond (13) comportant également plusieurs secteurs de fond lesquels sont articulés sur un anneau d'actionnement (17') et lesquels peuvent être déplacés en faisant glisser l'anneau d'actionnement (17') depuis la position de fermeture vers la position d'ouverture, **caractérisé en ce que** le tube (25) présente, pour chacun des anneaux de actionnement (17, 17'), une fente (28, 28') s'étendant en biais dans laquelle s'engage un ergot (27, 27') de l'anneau d'actionnement (17, 17') correspondant, que les deux fentes (28, 28') s'étendant en biais présentent une inclinaison dans le même sens, et que chacune des fentes (28, 28') s'étendant en biais se prolonge, à son extrémité se trouvant en face de l'extrémité concernée du cylindre tubulaire de curseur (11), par une fente (29, 29') s'étendant dans le sens de la circonférence du cylindre tubulaire, faisant en sorte que l'ouverture soit du couvercle (12) soit du fond (13) uniquement provoque le retrait, dans un sens différent selon le cas de figure, de l'élément de serrage (24) ou des éléments de serrage (24) du canal (23) concerné.

4. Curseur pour systèmes de transport pneumatique (110) selon la revendication 1, le couvercle (112) pouvant être pivoté autour d'un axe (116) parallèle à l'axe longitudinal du cylindre tubulaire de curseur (111), **caractérisé en ce que** l'élément de serrage (124) ou les éléments de serrage (124) sont montés sur un tube (125) qui se trouve en disposition coaxiale au sein du cylindre tubulaire de curseur (111), que le tube (125) peut être tourné autour de son axe longitudinal, et que la rotation du tube (125) est couplée au pivotement du couvercle (112).
